# EUROPEAN PATENT SPECIFICATION

(11) **EP 1 633 288 B1**
(45) Date of publication and mention of the grant of the patent: **21.05.2008**
(21) Application number: 04736743.8
(22) Date of filing: 14.06.2004
(51) Int. Cl.: A61F 2/30

(54) **DEVICE FOR PREVENTING DISLOCATION OF HIP ARTHROPLASTY IMPLANTS**
VORRICHTUNG ZUR VERHINDERUNG DER VERLAGERUNG VON HÜFTGELENKIMPLANTATEN
DISPOSITIF PERMETTANT D'EVITER LA LUXATION DE PROTHESES D'ARTHROPLASTIE DE HANCHE

(30) Priority: 17.06.2003 DK 200300894
(43) Date of publication of application: 15.03.2006
(73) Proprietor: Hopro A/S, 1260 Copenhagen K (DK)
(72) Inventor: HVOLRIS, Jesper, DK-4320 Lejre (DK); HANSEN, John, Bøgh, DK-4296 Nyrup (DK)
(74) Representative: Schouboe, Anne
(86) International application number: PCT/DK2004/000412
(87) International publication number: WO 2004/110317

(56) References cited:
- WO-A-00/57820
- DE-A- 3 741 490
- FR-A- 1 416 534
- US-A- 4 731 088
- US-A- 5 514 182

## Description

### FIELD OF THE INVENTION

The invention relates to a method and a device for preventing dislocation of hip arthroplasty implants. The invention prevents dislocation by providing a restraining force on the femoral part of the implant under all movements of the leg. The invention further provides a method for mounting the device on a hip arthroplasty implant.

### BACKGROUND OF THE INVENTION

In most western countries, the typical number of total hip joint arthroplasty every year lies between 0,5-1 ‰ of the population. World-wide an estimated number of 1 million people have a total hip joint arthroplasty every year (2003), with numbers Increasing. Although total hip joint arthroplasty is a very successful orthopaedic surgical procedure, it suffers from a serious drawback, namely dislocation of the hip joint. Dislocation typically occurs after a wrong movement of the leg by the patient, whereby the femoral head is drawn out of the cup. The causes are many, e.g. wrong or imprecise placement of the arthroplasty components, looseness in the surrounding tissue, of failure on the patients side to follow the restrictions in movements following from the surgery.

Dislocation after a total hip joint arthroplasty is a common adverse effect for patients having hip arthroplasty implants. Surveys report that 3-5 % of the patients experience a dislocation at some point. However, numbers as high as 15% has been reported. A dislocation can occur at any time in the lifetime of the arthroplasty, with an increased risk within the first month after the operation. It is not only a nuisance for the patient, it is also a substantial economical burden to hospitals and insurance companies.

A number of measures have been taken to avoid dislocation, including modification of the components to try and make the arthroplasty more stable. However, any use of locking mechanisms between the femoral ball and the acetabular socket has been fraught with problems. Such constrained sockets fall early because of high stresses arising from Impingement between the socket and femoral neck. This may cause the socket to pull out from the bone attachment in the pelvis, or dislodge the ring lock holding the devices together.

Collars for sealing joint prosthesis assemblies to prevent diffusion of e.g. debris or lubricants are well known from the prior art, e.g. FR 1416534, DE 3741490, US 4731088, US 5514182 or WO 00/57820. However, none of these provide any means for preventing dislocation of the joint prosthesis.

A number of flexible constraining devices have been suggested.

US 5,755,807 describes an implantable module for use with a prosthetic joint that utilises a bellows and bearing with a rotating seal for encapsulating the articulating members of the implant. The bellows and the bearing primarily serve the purpose of containing a lubricant to decrease the wear to the articulating parts. The implant module further serves the purpose of keeping the articulating bearing surfaces in proper alignment by providing a spring force from the metallic bellows. The bearing with the rotating seal connects the bellows and femoral neck of the implant. The rotating seal ensures free rotation of the femor in relation to the bellows.

US 6,228,122 discloses a semiconstrained net device to be attached to an acetabular cup and having a net ring around the femoral neck of the implant. The net device serves the purpose of preventing dislocation between the acetabular cup and the replacement femur. The net is fixed at specific positions of the acetabular cup to provide tension only at specific positions of the hip joint. In alternatively embodiments, the net is attached directly to a point on the femoral bone in one end and in the bone adjacent to the acetabular cup in the other end. This however, does not seem practically possible due to the difficulties that occur while placing the bore holes correct in the acetabular rim and hereby getting the correct tension of the semiconstraining device.

The net described in 6,228,122 is tightened to the cup of the adjacent bone by drawing a string of the net through a hole in the cup or bone. It is described that the tension of the net can be either pre-set or tensioned intraoperatively, presumably by knots on the string. It is a disadvantage of the pre-set tension that the tension of the device can not be precisely adapted to the anatomy of the patient. It is a disadvantage that the intraoperative tensioning can not be performed in a reproducible manner.

### SUMMARY OF THE INVENTION

In the creative intellectual process leading to the present invention, the inventors have applied their detailed expertise within the field of hip arthroplasty combined with a dynamical and mechanical approach. This has given a thorough understanding of the dynamical aspects of hip dislocation in arthroplasty implants.

It is a problem of the existing devises that they only serve as a stop for preventing the femoral head from popping out of the cup. They do not oppose movement to positions where such popping is likely to occur, and as the forces in play in these positions are extremely large, it is the experience of the inventors that they either allow dislocation or cannot endure the strain and break.

Dislocation may occur at specific positions of the joint and during special combinations of movements of the leg. Hence, in order for an anti-dislocation device to function properly, it should prevent the joint in reaching positions where dislocations are likely to occur by restraining movements typically leading to those positions. Alternatively the device should provide a further extra securing of the femoral head in the cup at the positions where dislocations are likely to occur. Both these demands may be met by a device which
a) provides a restraining force opposing movements typically leading to positions where dislocations are likely to occur, and which
b) provides an extra securing force for holding the femoral head in the cup when the leg is at positions where dislocations are likely to occur.

The device according to present invention provides these functions whilst representing a substitution of the anatomic and physiologic fibrous capsule. In order not to reduce the freedom of movement of the patient's leg, the restraining and securing forces should preferably increase with the amplitude of the movement.

In this description, a hip arthroplasty implant comprises at least the following components, an acetabular cup to be mounted in the acetabular cavity of a pelvis, a femoral stem to be mounted in the proximal end of a femoral bone and having a femoral neck, and a femoral head to be mounted on the femoral neck and to be situated in a receiving cavity of the acetabular cup. These components will be referred to throughout the text.

In general, any movement of a physical object can be resolved into a translation of the object to obtain its new position followed by a rotation around a properly chosen axis to obtain its new orientation. In a similar fashion, any normal movement of a hip joint can be resolved into two rotations around perpendicular axes; a rotation around a first axis through the centre of the femoral neck followed by a rotation around a second axis through the centre of the femoral head, which axis is perpendicular to the first axis. In the present specification, rotation around the first axis is referred to as axial rotation, whereas rotation around the second axis is referred to as planar rotations (as the femoral neck spans a plane during this). Almost all movements of the leg include combinations of axial and planar rotations. The above description of hip joint movements is by no means the only possible choice, but serves to unambiguously define the scope of the present invention and can be used to describe all possible hip joint movements.

The invention provides a device for preventing dislocation of a hip arthroplasty implant as defined in claim 1.

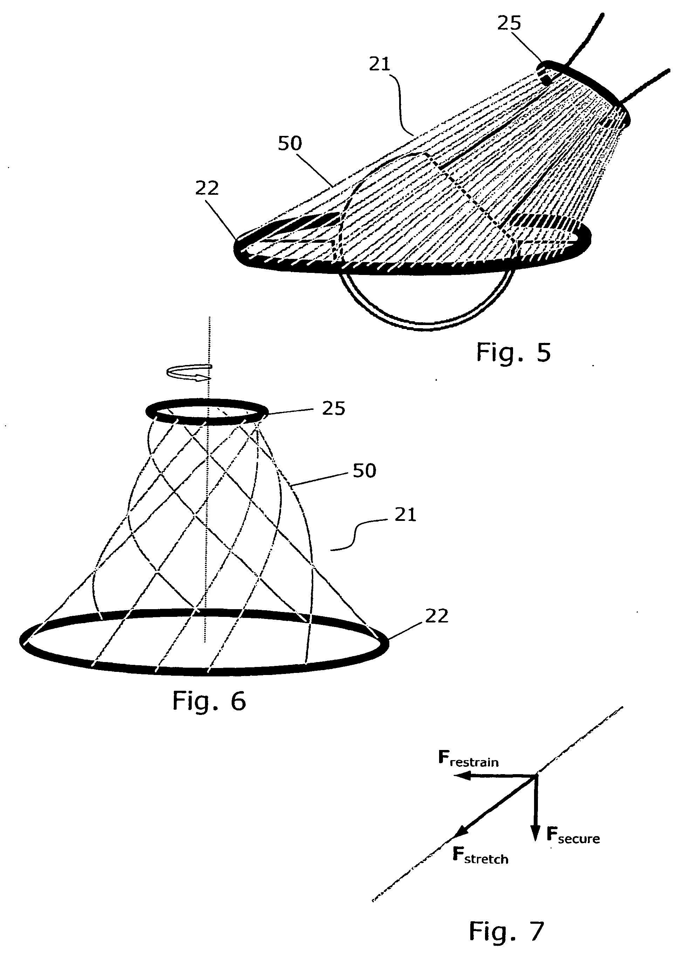

It is of importance that the second rim is fastened in fixed relation to the femoral neck and that it is fastened to at least partly circumvent the femoral neck. The fixed fastening means that the femoral neck can not rotate without twisting the collar. Thus, any movement involving axial rotations will twist the tubular collar and thereby also stretch it along its longitudinal axis. Thereby, the tubular collar will execute a force restraining the movement of the femoral neck and a force pulling the femoral head towards the receiving cavity of the cup. These forces arise because the longitudinal stretching generates a force which can be resolved into two perpendicular components. A first component is perpendicular to the neck and represents a force restraining the movement. A second component is parallel to the neck and represents a securing force for holding the femoral head in the cup (please refer to Figure 7). The twisting of the collar has another important property. If the movement involves only planar rotations, the collar is stretched in one side and slacked in the opposite side. However, as most movements of the leg involve both axial and planar rotations, the collar will be stretched/slacked and twisted at the same time. The twisting of the collar will tighten the slacked side of the collar whereby this part will also contribute with a securing force component. In the devices described in the prior art, the femoral neck can rotate freely, and the net/bellows are not twisted. Therefore, it is an important feature that the second rim is fastened n fixed relation to the femoral neck.

As the forces work on the femoral neck, and not the head itself, it is important that they work on all sides of the neck at the same time. If not, the total resulting securing force component may be asymmetric resulting in a skew pull of the neck towards the cup. Therefore, it is an important feature that the second rim is fastened so as to at least partly circumvent the femoral neck. Preferably, the tubular collar, the rims and the fastenings means completely encircle the neck etc., however, for the purpose of mounting or assembling the device, it may be advantageous to have small open or incomplete sections.

The tubular collar may be a mesh woven in the form of an open stocking by e.g. crosslinked HMDPE fibres, polyethylene fibres, or fibres of other known biocompatible materials. The fabric of the mesh preferably consists of bioacceptable material. To provide the longitudinal stretching force, the tubular collar is preferably elastic in at least a longitudinal direction. The elasticity should be chosen so that the collar is stretched without tearing by the forces in play when the device is mounted on a grown up human being. Further, the tubular collar may be elastic in a radial direction. The elastic properties of the mesh are related to the weaving technique used in the fabric.

In alternative embodiments, the tubular collar may be formed by a sheet or membrane of known, elastic biocompatible material such as artificial rubbers. The elastic material is preferably enforced to increase it strength and/or elasticity and/or providing a maximum stretching limit for the material. The tubular collar may also incorporate a bellows comprising metal springs. In all embodiments, the tubular collar preferably has the shape of a truncated cone.

The first and second fastening means preferably comprise first and second rings attached to the first and the second rim. The first ring may have one or more protrusions on, or incisions in, a first surface so that it can be assembled with a surface part of the acetabular cup, or with a flange to be fixed on the acetabular cup, having at least one corresponding incision or protrusion. There exist a large number of possible designs for attaching the first rim to the acetabular cup, all of which provide the essential feature of easy fixation using only biocompatible materials.

In a preferred embodiment, the second ring attached to the second rim has a slot for receiving a clamp formed as an open ring to be inserted in said slot. The femoral neck, or a flange to be fixed on the femoral neck, has an outer circumference with a shape corresponding to a shape of the inner circumference of said clamp. By mounting the second ring on the femoral neck (or the flange), the clamp can be inserted in the slot to fix the ring on the femoral neck.

Alternatively, the second ring may have one or more protrusions on or incisions in an internal circumference so that it can be mounted on the femoral neck, or a flange to be fixed on the femoral neck, having at least one corresponding incision or protrusion around its outer circumference.

This will allow for each of the end of the tubular collar to be fixed at several different positions so that the collar can be mounted in a position where it is not twisted.

In an alternative embodiment, the second fastening means comprises a ring attached to the second rim, which ring can be fixedly mounted around the femoral neck by shrink fitting. Alternatively, the second fastening means comprises a ring attached to the second rim, which ring can be fixedly mounted around the femoral neck by at least one bolt, screw, clamp or clip. There exist a large number of possible designs for attaching the second rim to the femoral neck, all of which provide the essential feature of prevent longitudinal and rotational movement of the second rim along/around the femoral neck.

The movements of the joint away from the neutral position may be described by the previously defined axial and planar rotations. Alternatively, the movements may be described by the movements of the leg using the anatomical terms from the following definitions:
- Sagittal Plane: A vertical plane extending in an antero-posterior direction dividing the body into right and left parts.
- Frontal Plane: A vertical plane at right angles to the sagittal plane that divides the body into anterior and posterior portions.
- Transverse Plane: A transverse plane at right angle to the long axis of the body, same as horizontal plane.
- Extension: A movement at a joint resulting in separation of two ventral surfaces (as opposed to flexion).
- Flexion movement: A movement at a joint resulting in approximation of two ventral surfaces (as opposed to extension).
- Abduction: A movement from a midline (as opposed to adduction).
- Adduction: A movement toward a midline (as opposed to abduction).

The movement of the leg may be described by the following movements:
- flexion movement in a sagittal plane,
- extension movement in a sagittal plane,
- adduction movement in a frontal plane,
- abduction movement in a frontal plane,
- external rotation in a transverse plane,
- internal rotation in a transverse plane,
or any combination thereof.

In the experience of the inventors, dislocation is especially likely to occur under movements that are similar but inverse to those used by surgeons to relocate dislocated joints. These may be described as a combination of flexion and inward rotation and perhaps adduction.

The forces in play under movements of the joint of the hip implant will be described in larger detail elsewhere in the specification.

The position of the femoral component during mounting of the device is important for the performance. Preferably, the tubular collar should be uniformly tight when the leg is in a neutral position, so as to act upon movements towards positions where dislocations are likely to occur. However, the leg has many natural positions (when the patient is standing, lying, sitting etc.), and an appropriate position should be chosen. When an appropriate neutral position is chosen, attention should be paid to acetabular inclination and anteversion and leg length, among other things.

The first and second fastening means preferably comprise first and second rings attached to the first and the second rim. These rings are to be attached at the acetabular cup and at the femoral neck, respectively. As the femoral head is larger that the circumference of the second ring, the second ring is preferably mounted on the femoral neck before putting on the femoral head. Optionally, only the second ring is mounted, in which case the collar can be fastened to the second ring at a later stage.

The device and methods according to the invention is adapted to be used for hip arthoplasty in humans, but may also be applicable for dogs, horses, or other mammals.

### BRIEF DESCRIPTION OF THE DRAWINGS

Figure 1 shows a hip with a typical hip arthroplasty implant.
Figure 2 is an illustration of a hip arthroplasty implant mounted with a device for preventing dislocating according to a preferred embodiment of the invention.
Figure 3A and B illustrates the first fastening means for fastening the device according to the invention to the acetabular cup.
Figure 4A, B, and C illustrates the second fastening means for fastening the device according to the invention to the femoral stem.
Figure 5 shown a planar rotation movement of a hip arthroplasty implant with a device for preventing dislocating according to the invention.
Figure 6 shown an axial rotation movement of a hip arthroplasty implant with a device for preventing dislocating according to the invention.
Figure 7 illustrates the forces from the tubular collar of the device under the movements illustrated in Figures 5 and 6.
Figure 8 shows an embodiment of the tubular collar of the device according to the invention.
Figure 9 is a graph showing the response of a spring and a rubber band.

### DETAILED DESCRIPTION OF THE INVENTION

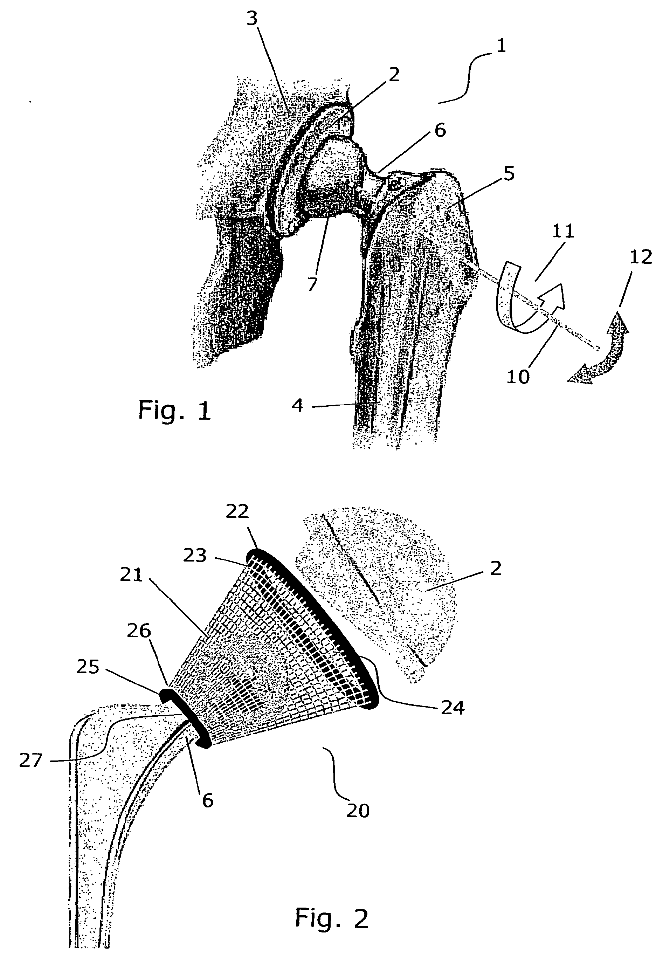

Figure 1 shows a typical hip arthroplasty implant 1 with an acetabular cup 2 cemented in the acetabular cavity of the human pelvis 3, a femoral stem 4 mounted in the proximal end of a human femoral bone 5, the femoral stem having a femoral neck 6, and a femoral head 7 mounted on the femoral neck 6 and situated in a receiving cavity of the acetabular cup 2.

Also shown in Figure 1 is the first axis 10 of the femoral neck 6. Arrow 11 illustrates axial rotations while arrow 12 illustrates planar rotation according to the previous definitions.

Figure 2 shows an embodiment of the device 20 for preventing dislocation according to the present invention. The device 20 has a tubular collar 21 made from a woven mesh. The device 20 has a first fastening means 22 to fasten a first rim 23 of a first open end 24 of the collar 21 to the acetabular cup 2. Similarly, the device 20 has a second fastening means 25 to fasten a second rim 26 of a second open end 27 of the collar 21 to the femoral neck 6.

The fastening means 22 and 25 provides the fixation of the collar to the cup and the stem. The collar can in principle be fastened to either the cup or stem prior to the operation. However, typically the collar can cause inconvenience during mounting of the stem and cup in the patient. For this reason, the mounting and final fastening of the collar will preferably be done at the end of the hip arthroplasty operation. During the final fastening, the tubular collar should be at least substantially uniformly tight and straight (little or no twisting) with the leg in a neutral position. It is therefore important that the fastening means can fix the collar at several positions around the first axis 10.

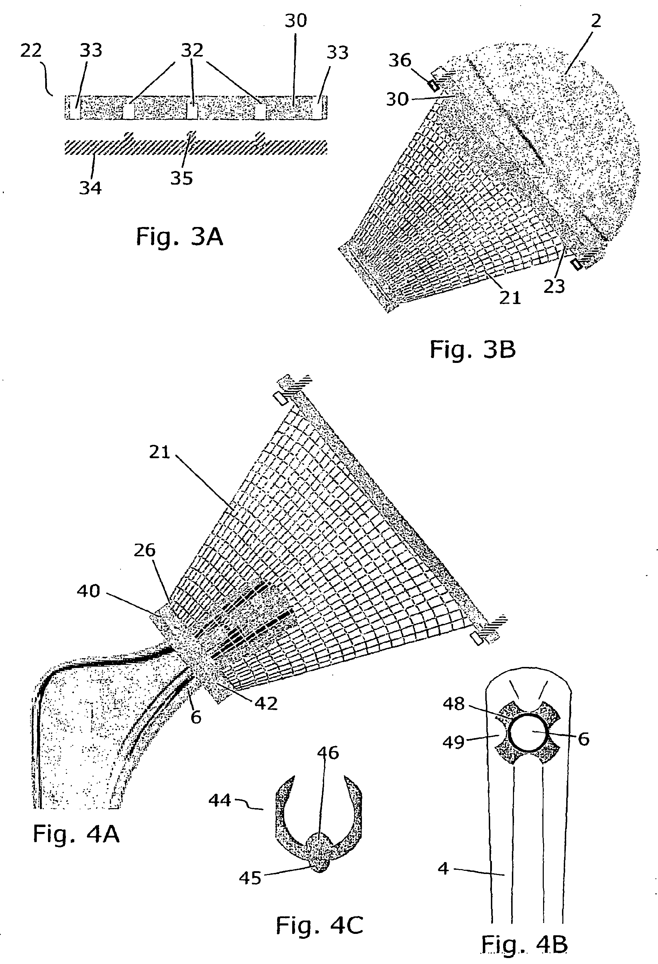

Figure 3A and B illustrates a preferred embodiment of the first fastening means 22. In Figure 3A, the first fastening means 22 consist of a ring 30 attached to the first, wider rim 23 of the tubular collar 21 (see Figure 3B). The ring 30 has a number of holes or incisions 32 to fit a number of protrusions 35 on a part 34 of the acetabular cup 2 facing the ring 30. The incisions 32 and protrusions enables the ring 30, and thereby the collar 21, to be securely fastened in a number of different positions around the first axis 10 (see Figure 1).

The ring 30 further has two or more holes 33 for screws or bolts to fix the ring 30 to the part 34. Figure 3B illustrates the ring 30 fastened on the acetabular cup 2 with screws 36.

Figure 4A-C illustrates a preferred embodiment or the second fastening means 25. The second fastening 25 consist of a ring 40 attacked to the second, narrower rim 26 of the tubular collar 21. The ring 40 is to be fastened on the femoral neck 6. As shown in Figure 4B, a stationary flange 48 is fixated on the femoral neck 6. The flange 48 has incisions 49 representing the positions around the first axis 10 (see Figure 1) on which the collar 21 can be fastened. To fasten the ring 40 on the flange 48, the ring 40 is slit over the flange 48 at the desired position around the first axis. The second fastening also includes a locking clamp 44 shown in Figure 4C. The clamp 44 fits in a slot 42 in the ring 40 (see Figure 4A). The straight side parts of the clamp 44 fits the width of the slot 42, so that the clamp can be inserted in the slot without clearance. An outer perimeter of the flange 48 of Figure 4B fits an inner perimeter of the clamp 44. The clamp has a protrusion 46 on its inner perimeter corresponding to the incisions 49 in the flange. The clamp 44 also has a pin 45 for inserting and removing the clamp. The ring is locked in the desired positions by insertion of the clamp 44 in the slot 42.

When the joint of the implant undergoes planar rotations, one side of the collar is stretched as illustrated in Figure 5. The side of the collar opposite to the stretched side will be slacked. If the collar is a mesh as shown in Figure 5, then stretching the collar means stretching individual strings in the mesh. As illustrated in Figure 7, the stretching of a string produces a stretching force F_{stretch} having a component Fᵣₑₛₜᵣₐᵢₙ restraining the planar rotation and component F_{secure} pulling the femur towards the pelvis and thereby securing the femoral head in the cup. The same forces come into play if a material such as an artificial rubber sheet forms the collar.

As described previously, axial rotations as illustrated in Figure 6 will twist the tubular collar because the femoral neck can not rotate without the collar. As the distance between the first and second fastening means 22 and 25 does not change, the twisting stretches the individual strings in the mesh, and thereby also the collar along its longitudinal axis. Thus, the forces of Figure 7 come into play as for the planar rotation of Figure 5.

When the movement is a combination of planar and axial rotations, as most normal movements are, the stretching/slacking of Figure 5 is combined with the twisting or Figure 6. Thus, the swings on both the stretched and the stacked side of Figure 5 will be stretched due to the twisting. Thereby, the strings an the slacked side will not be slacked, only less stretched than the strings on the stretched side. The force components Fᵣₑₛₜᵣₐᵢₙ and F_{secure} of Figure 7 will increase due to this combined effect, so that
- the femoral head will be held even more securely in the cup, and
- the motion will be restrained even further, minimising the risk for the leg to move to a dislocating position.

Thus, the twisting of the collar enhances the anti-dislocation effect of the device.

As mentioned previously, the restraining and securing forces provided by the collar may increase with the amplitude of the movement. This will increase the freedom of movement of the leg for small amplitudes while increasing the restraining and securing forces for large amplitudes. The response of the collar depends on its elasticity, which depends on the applied material and the weave of the mesh.

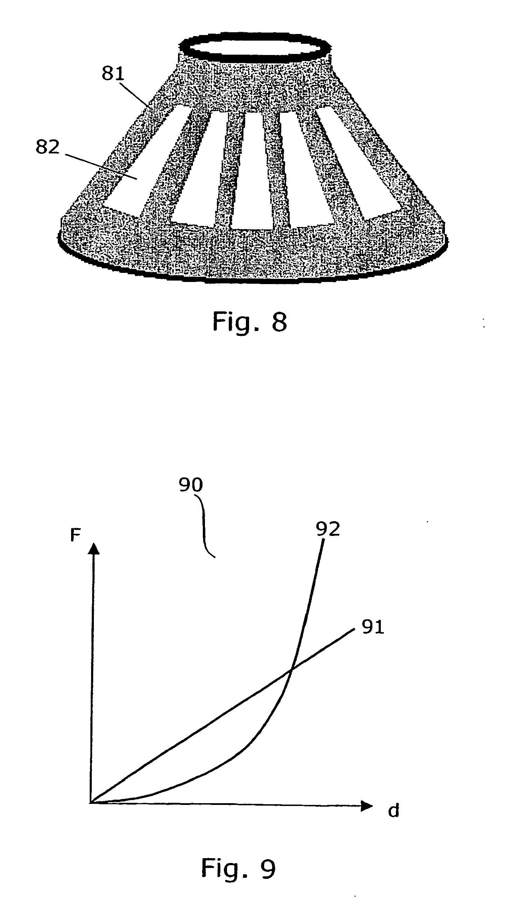

The tubular collar of the device according to the invention may be embodied in many different ways, all providing the essential features. A large number of designs and materials may be applied. Figure 8 shows another design of a tubular collar 81 made from an enforced artificial rubber tubing with openings 82 for improving the mobility of the material upon axial rotations. The artificial rubber tubing may have varying thickness or material properties to increase mobility.

The spring force from a typical metal spring will increase linearly with the distance in most of its dynamical range. This is illustrated by the line 91 in the graph 90 of Figure 9 having the spring/elastic force F along the principal axis and the distance d along the secondary axis, some deviation from the line 91 will occur when the spring is close to fully stretched. Elastic materials such as e.g. artificial rubber have a different response. Here, the force increases nonlinearly with the distance of extension. Curve 92 in Figure 9 shows the response of a normal rubber band.

In a preferred embodiment, the tubular collar response with a force which increase nonlinearly with an amplitude of a movement of the femoral neck. In another embodiment, the tubular collar response with a force which increase at least substantially linearly with an amplitude of a movement of the femoral neck.

## Claims

1. A device for preventing dislocation of a hip arthroplasty implant (20), the hip arthroplasty implant comprising an acetabular cup (2) to be mounted in the acetabular cavity of a pelvis (3), a femoral stem (4) to be mounted in the proximal end of a femoral bone (5) and having a femoral neck (6), and a femoral head (7) to be mounted on the femoral neck and to be situated in a receiving cavity of the acetabular cup,
the device comprising
- a tubular collar (21) for executing a restraining force opposing movements of the femoral bone leading to positions where dislocations can occur, the tubular collar having a first end (24) and a second end (27),
- first fastening means (22) for fastening the first end in fixed relation to and at least partly encircling the receiving cavity of the acetabular cup, and
- second fastening means (25) for fastening the second end in fixed relation to and at least partly circumventing the femoral neck to prevent longitudinal movement of the second end along the femoral neck and rotational movement of the second end around the femoral neck, **characterised in that** the tubular collar is formed in an elastic material with openings.

2. The device according to any of the preceding claims, wherein the tubular collar is elastic in at least a longitudinal direction.

3. The device according to any of the preceding claims, wherein the tubular collar is elastic in at least a radial direction.

4. The device according to any of the preceding claims, wherein the tubular collar is an elastic mesh.

5. The device according to any of the preceding claims, wherein the first fastening means comprises a ring (30) attached to the first end, the ring having one or more protrusions on or incisions (32) in a first surface, and wherein an accessible surface part (34) of the acetabular cup, or of a flange to be fixed on the acetabular cup, has at least one corresponding incision or protrusion (35).

6. The device according to any of the preceding claims, wherein the second fastening means comprises a ring (40) attached to the second end, the ring having one or more protrusions on or incisions in an internal circumference, and wherein the femoral neck or a flange (48) to be fixed on the femoral neck has at least one corresponding incision or protrusion (49) around its outer circumference.

7. The device according to any of claims 1 - 5, wherein the second fastening means comprises a ring (40) attached to the second end, the ring having a slot (42), and a clamp (44) formed as an open ring to be inserted in said slot, and wherein the femoral neck or a flange (48) to be fixed on the femoral neck, has an outer circumference with a shape corresponding to a shape of the inner circumference of said clamp.

## Patentansprüche

1. Vorrichtung zur Verhinderung einer Dislokation einer Hüftgelenkprothese (20), wobei die Hüftgelenkprothese eine Pfanne (2), die in die Pfannenschale eines Beckens (3) eingesetzt wird, einen Oberschenkelschaft (4), der in das proximale Ende eines Oberschenkelknochens (5) eingesetzt wird und einen Oberschenkelhals (6) aufweist, und einen Oberschenkelkopf (7), der auf den Oberschenkelhals aufgesetzt wird und in einer aufnehmenden Kavität der Pfanne anzuordnen ist, aufweist,
wobei die Vorrichtung umfasst
- einen schlauchartigen Kragen (21) zum Ausüben einer Festhaltekraft, die Bewegungen des Oberschenkelknochens entgegenwirkt, die zu Positionen führen, bei denen Dislokationen auftreten können, wobei der schlauchartige Kragen ein erstes Ende (24) und ein zweites Ende (27) aufweist,
- ein erstes Befestigungsmittel (22) zum Befestigen des ersten Endes in einer starren Beziehung zur aufnehmenden Kavität der Pfanne, wobei diese wenigstens teilweise umschlossen wird, und
- ein zweites Befestigungsmittel (25) zum Befestigen des zweiten Endes in einer starren Beziehung zum Oberschenkelhals, wobei dieser wenigstens teilweise umgangen wird, um Längsbewegungen des zweiten Endes entlang des Oberschenkelhalses und Rotationsbewegungen des zweiten Endes um den Oberschenkelhals zu verhindern, **dadurch gekennzeichnet, dass** der schlauchartige Kragen aus einem elastischen Material mit Öffnungen hergestellt ist.

2. Vorrichtung nach einem der vorhergehenden Ansprüche, wobei der schlauchartige Kragen wenigstens in einer Längsrichtung elastisch ist.

3. Vorrichtung nach einem der vorhergehenden Ansprüche, wobei der schlauchartige Kragen wenigstens in einer Radialrichtung elastisch ist.

4. Vorrichtung nach einem der vorhergehenden Ansprüche, wobei der schlauchartige Kragen ein elastisches Netz ist.

5. Vorrichtung nach einem der vorhergehenden Ansprüche, wobei das erste Befestigungsmittel einen Ring (30) umfasst, der an dem ersten Ende befestigt ist, wobei der Ring einen oder mehrere Vorsprünge auf oder Einkerbungen (32) in einer ersten Fläche aufweist, und wobei ein zugänglicher Flächenteil (34) der Pfanne, oder eines an der Pfanne zu befestigenden Flansches, wenigstens eine entsprechende Einkerbung oder einen entsprechenden Vorsprung (35) aufweist.

6. Vorrichtung nach einem der vorhergehenden Ansprüche, wobei das zweite Befestigungsmittel einen Ring (40) umfasst, der an dem zweiten Ende befestigt ist, wobei der Ring einen oder mehrere Vorsprünge auf oder Einkerbungen (32) in einem inneren Umfang aufweist, und wobei der Oberschenkelhals oder ein am Oberschenkelhals zu befestigender Flansch (48) wenigstens eine entsprechende Einkerbung oder einen entsprechenden Vorsprung (49) aufweist.

7. Vorrichtung nach einem der Ansprüche 1-5, wobei das zweite Befestigungsmittel einen Ring (40), der an dem zweiten Ende befestigt ist, wobei der Ring einen Schlitz (42) aufweist, und eine Klemme (44), die als offener Ring zum Einsetzen in den Schlitz ausgebildet ist, umfasst, und wobei der Oberschenkelhals oder ein am Oberschenkelhals zu befestigender Flansch (48) einen äußeren Umfang in einer Form aufweist, die der Form des inneren Umfangs der Klemme entspricht.

## Revendications

1. Dispositif destiné à prévenir le descellement d'une prothèse d'arthroplastie de la hanche (20), ladite prothèse d'arthroplastie de la hanche comportant une cupule cotyloïdienne (2) devant être installée dans la cavité cotyloïde du bassin (3), une tige fémorale (4) devant être installée dans l'extrémité proximale de l'os fémoral (5) et possédant un col fémoral (6), et une tête fémorale (7) devant être installée sur le col fémoral et devant être mise en place dans une cavité réceptrice de la cupule cotyloïdienne,
ledit dispositif comportant
- un manchon tubulaire (21) destiné à créer une force de retenue qui s'oppose aux mouvements de l'os fémoral conduisant à des positions dans lesquelles des descellements peuvent se produire, ledit manchon tubulaire comportant une première extrémité (24) et une deuxième extrémité (27),
- un premier moyen de fixation (22) destiné à fixer la première extrémité de manière fixe dans la cavité réceptrice de la cupule cotyloïdienne, en entourant au moins en partie ladite cavité, et
- un deuxième moyen de fixation (25) destiné à fixer la deuxième extrémité de manière fixe sur le col fémoral, de manière à circonvenir au moins en partie ledit col afin d'empêcher le déplacement longitudinal de la deuxième extrémité le long du col fémoral et la rotation de la deuxième extrémité autour du col fémoral, **caractérisé en ce que** le manchon tubulaire est constitué d'un matériau élastique comportant des ouvertures.

2. Dispositif selon l'une quelconque des revendications précédentes, dans lequel le manchon tubulaire est élastique dans au moins une direction longitudinale.

3. Dispositif selon l'une quelconque des revendications précédentes, dans lequel le manchon tubulaire est élastique dans au moins une direction radiale.

4. Dispositif selon l'une quelconque des revendications précédentes, dans lequel le manchon tubulaire est constitué d'un treillis élastique.

5. Dispositif selon l'une quelconque des revendications précédentes, dans lequel le premier moyen de fixation comporte une bague (30) montée sur la première extrémité, cette bague comportant une ou plusieurs protubérances ou incisions (32) sur une première surface, et dans lequel une pièce de surface accessible (34) de la cupule cotyloïdienne, ou une collerette devant être fixée sur la cupule cotyloïdienne, possède au moins une incision ou protubérance correspondante (35).

6. Dispositif selon l'une quelconque des revendications précédentes, dans lequel le deuxième moyen de fixation comporte une bague (40) montée sur la deuxième extrémité, cette bague comportant une ou plusieurs protubérances ou incisions au niveau d'une circonférence interne, et dans lequel le col fémoral ou une collerette (48) devant être fixée sur le col fémoral possède au moins une incision ou protubérance correspondante (49) autour de sa circonférence externe.

7. Dispositif selon l'une quelconque des revendications 1 à 5, dans lequel le deuxième moyen de fixation comporte une bague (40) montée sur la deuxième extrémité, cette bague comportant une rainure (42), et un collier (44) en forme de bague ouverte devant être inséré dans ladite rainure, et dans lequel le col fémoral ou une collerette (48) devant être fixée sur le col fémoral présente une circonférence externe dont la forme correspond à la forme de la circonférence interne dudit collier.
